# EUROPEAN PATENT APPLICATION

(11) **EP 0 748 870 A2**
(43) Date of publication of application: **18.12.1996**
(21) Application number: 96109045.3
(22) Date of filing: 05.06.1996
(51) Int. Cl.: C12N 15/19, A61K 48/00, C12N 15/86

(54) **Oncostatin M induced hematopoiesis**

(30) Priority: 06.06.1995 US 473026
(71) Applicant: Bristol-Myers Squibb Company, New York, N.Y. 10154 (US)
(72) Inventor: Clegg, Christopher H., Seattle, WA 98177 (US)
(74) Representative: Kinzebach, Werner, Dr.

(57) **Abstract**

The present invention relates to a method of inducing proliferation and differentiation of mammalian hematopoietic stem cells by transfection and expression of the oncostatin M gene. The method also relates to a method of increasing the survival of lymphocytes exposed to cytotoxic agents or radiation by expressing oncostatin M in transfected hematopoietic stem cells.

## Description

### BACKGROUND OF THE INVENTION

Of the various cells generated during hematopoiesis, none is believed to have the ability to self-regenerate and maintain its pluripotent potential as is characteristic of the hematopoietic stem cell. This regenerative and pluripotent ability permits complete restoration of hematopoietic function following transplantation of fractional amounts of bone marrow. Due to this capacity to self-regenerate, hematopoietic stem cells are also ideally suited for genetic modifications that survive the life of the host.

Hematopoietic stem cells differentiate along one of two pathways to give rise to either myeloid or lymphoid cell lineages. Whereas myeloid stem cells differentiate into macrophages, eosinophils, erythrocytes, megakaryocytes, and mast cells, the lymphoid precursors give rise to B cells and T cells. Mature B lymphocytes originate primarily in the bone marrow of adult mammals. T lymphocytes originate as precursors in bone marrow but undergo differentiation in the thymus. Upon entry into the thymus, T-cell precursors lack the critical markers that characterize mature T cells; that is, they generally do not express on their surfaces TCR molecules, the CD3 complex, or the CD4 or CD8 molecules. Because these cells lack both CD4 and CD8, they are often referred to as "double-negative" cells. Thymocytes develop from this double negative pool into cells that are both CD4⁺ and CD8⁺, referred to as "double-positive" cells. In turn, double-positive cells further differentiate into relatively mature thymocytes that express either CD4 or CD8 and high levels of the TCR-CD3 complex.

Given the central role of stem cells in hematopoietic development, what is needed in the art is a method to induce hematopoietic regeneration, proliferation and differentiation. Stimulating hematopoiesis would provide opportunities for therapeutic intervention in a wide variety of disorders resulting from deficiencies in myeloid and lymphoid cells as well as targeted genetic therapies. Quite surprisingly, the present invention fulfills these and other related needs.

### SUMMARY OF THE INVENTION

The present invention provides a method of inducing proliferation and differentiation of mammalian hematopoietic stem cells by genetic modification. The method comprises transfecting the mammalian hematopoietic stem cells with a vector comprising a gene coding for oncostatin M, expressing the gene to produce an effective amount of oncostatin M, and growing the stem cells in a medium supporting hematopoietic proliferation and differentiation.

In one embodiment, the cells are grown in a histocompatible mammalian host, such as in human bone marrow. In another embodiment, the cells are grown in a culture medium. The gene for oncostatin M may be under the control of a constitutive or tissue specific promoter. In a preferred embodiment, the gene coding for oncostatin M is under control of the proximal promoter from the lck gene. The hematopoietic stem cells may also comprise a vector expressing a rearranged T cell receptor or immunoglobulin.

In another aspect, the present invention is directed to a method of increasing the survival of lymphocytes. The method comprises transfecting hematopoietic stem cells with a gene coding for oncostatin M and expressing the gene to induce hematopoiesis within a mammal. Induction of hematopoiesis within the mammal increases the survival of the mammal's lymphocytes when exposed to a cytotoxic agent such as radiation or a chemotherapeutic, such as mitomycin C. The lymphocytes which demonstrate increased survival may be non-transfected lymphocytes indigenous to the mammal, or derived from differentiation of the transfected hematopoietic stem cell.

The methods of the present invention are useful in the treatment of such diseases as lymphoma, aplastic anemia, β-thalassemia, thrombocytopenia, hemophilia, combined immunodeficiency, AIDS, or leukemia. Further, the methods of the present invention are useful for increasing the survival of lymphocytes during radiation therapy or chemotherapy.

### BRIEF DESCRIPTION OF THE DRAWINGS

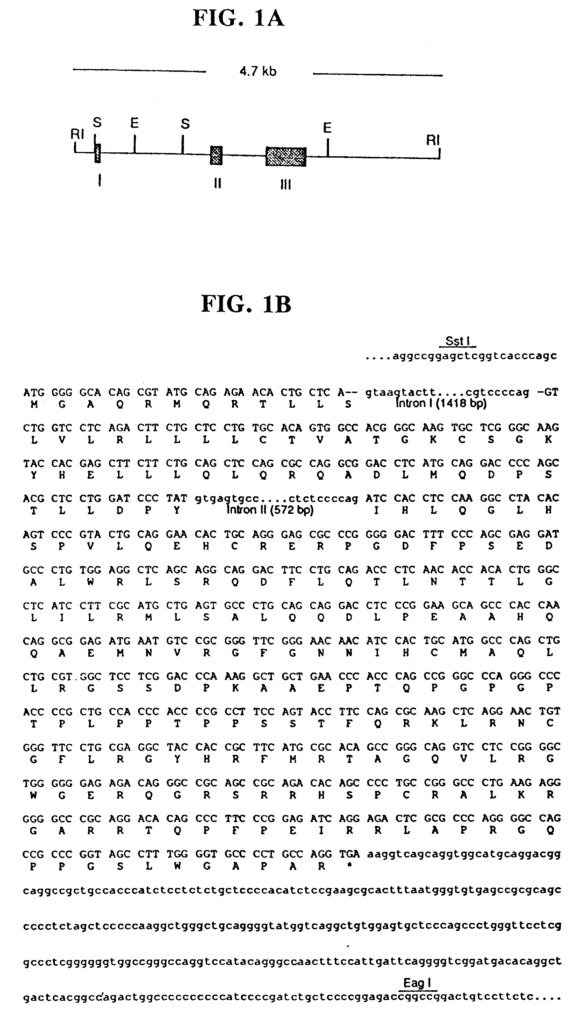

Figs. 1A and 1B show the bovine oncostatin M gene. (A) Partial restriction endonuclease map of bovine genomic DNA (4.7 kb). RI, *Eco RI*; *S*, *Sst I*; E, *Eag I*. (B) Open reading frame of the bOSM gene. This DNA sequence includes the exon/intron borders and the flanking regions that are included in the bOSM expression vectors.

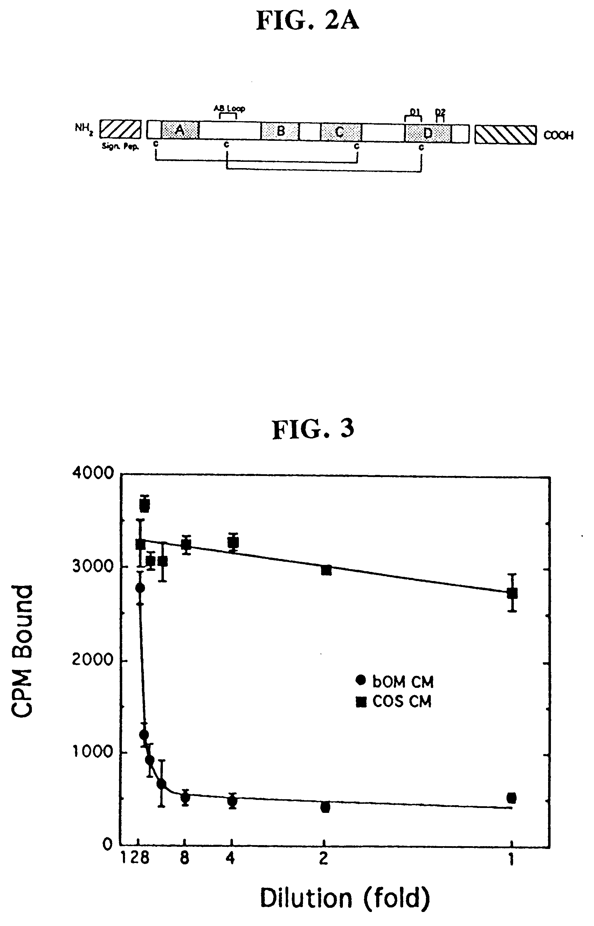

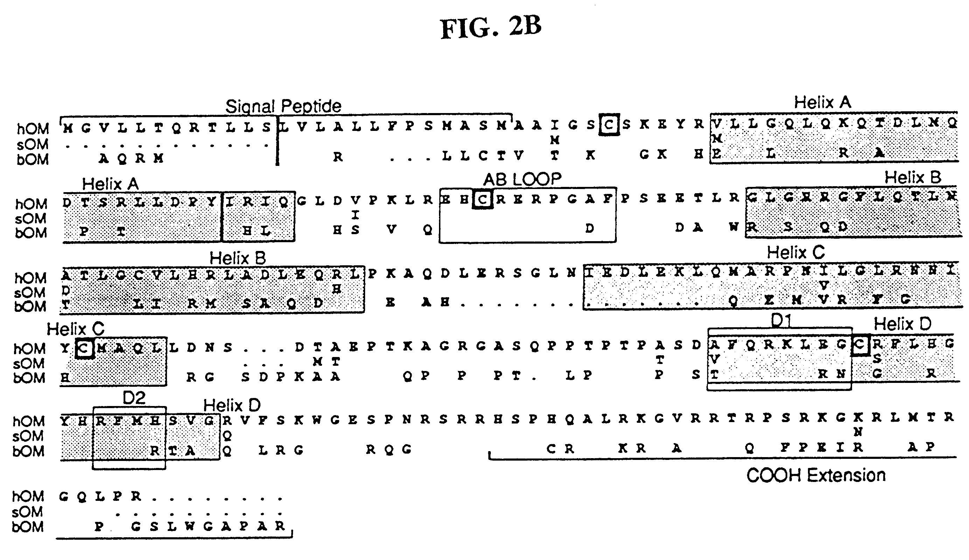

Figs. 2A and 2B collectively shows oncostatin M structural regions and alignment. (A) shows the structural domains of human oncostatin M (hOSM) protein. Shaded areas indicate the location of the four amphipathic helices. The hatched regions represent portions of the precursor protein that are processed by proteolysis. The cystein residues and intramolecular disulfide bonds are indicated. The AB loop and the D1 and D2 regions have been implicated in receptor binding. (B) shows a best-fit alignment of human OSM, simian OSM, and bovine OSM precursor proteins. Differences in amino acid sequence relative to the human sequences are indicated. Deletions in primary sequence are indicated by a dot. The two heavy lines indicate the location of the intron/exon boundaries in the human, simian, and bovine genes. Cysteine residues involved in disulfide bonding are shown in black boxes.

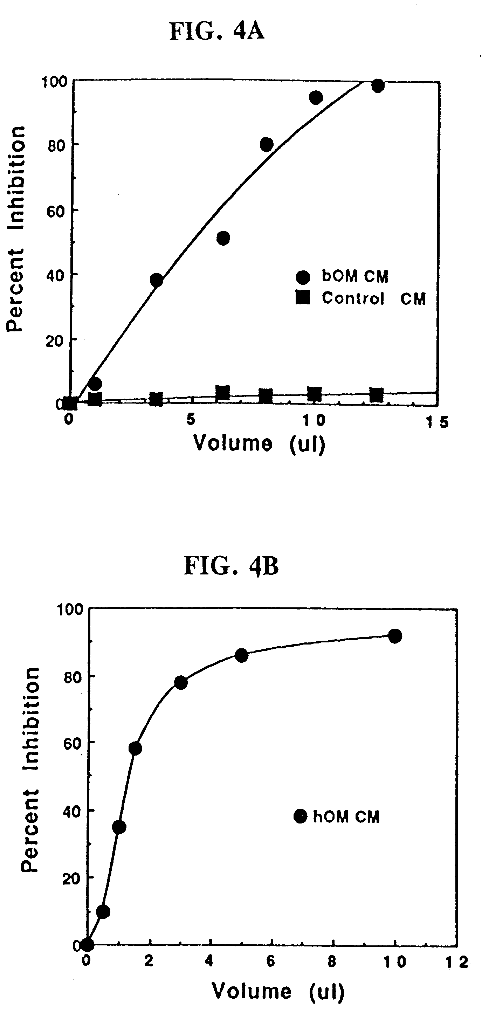

Fig. 3 shows inhibition of [¹²⁵I] human OSM binding to H2981 lung carcinoma cells by bovine OSM protein. Conditioned medium from COS cells transfected with a bovine OSM expression vector was serially diluted in binding buffer and incubated with cells for 1.5 h at 20° C. The cells were then rinsed and assayed for [¹²⁵I) human OSM binding.

Figs. 4A and 4B collectively show inhibition of human A375 melanoma cell proliferation by bovine OSM. (A) The indicated volumes of conditioned medium (CM) from COS cells transfected with either pπH3bOSM or pπH3M were diluted to a final volume of 200 µl with fresh culture medium and incubated with cells for 72 h. Cultures were pulsed with ¹²⁵I-deoxyuridine for 8 h. and then assayed for radionucleotide incorporation into DNA. (B) A similar experiment performed with conditioned medium isolated from COS cells transfected with the human OSM plasmid pπH3hOSM.

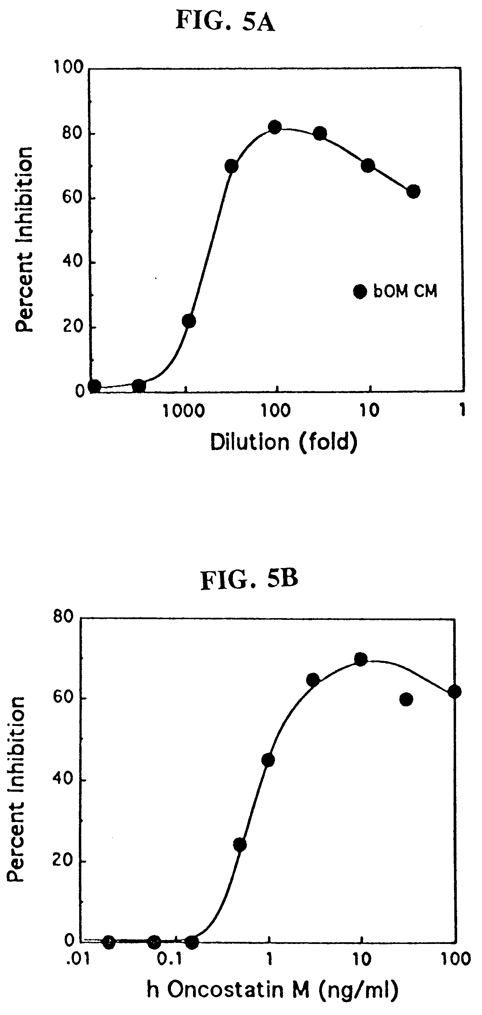

Figs. 5A and 5B collectively show inhibition of mouse M1 leukemia cell proliferation with bovine OSM. (A) The indicated volumes of conditioned medium (CM) from bOSM-transfected COS cells were diluted to a final volume of 200 µl with fresh culture medium and incubated with cells in microtiter plates. After 72 h., cultures were pulsed with [³H]thymidine for 6 h. and then assayed for radionucleotide incorporation into DNA. (B) Replicate conditions with pure recombinant human OSM protein.

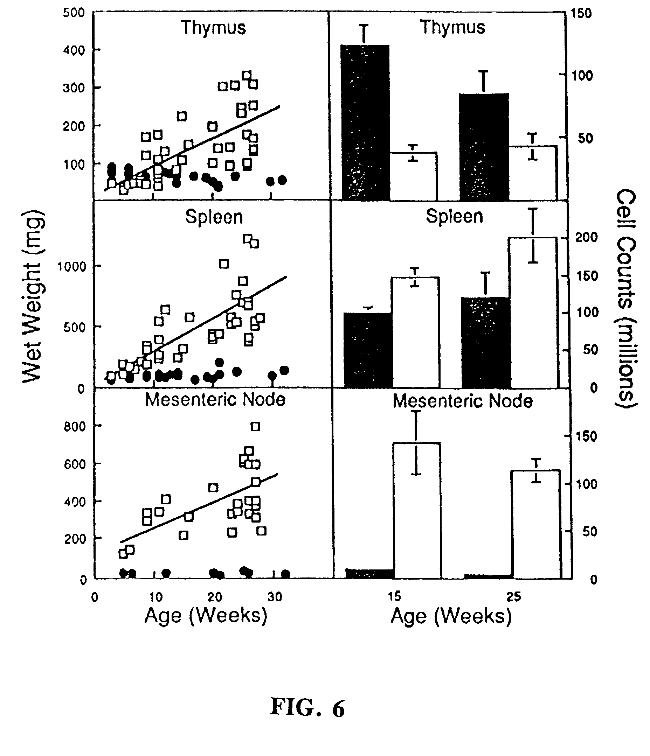

Fig. 6 shows alterations in wet weight and cell number in the lymphoid tissues of LckbOSM transgenic mice. Closed circles represent weight of thymus, spleen and mesenteric node from non-transgenic animals as a function of age. Open squares correspond to LckbOSM mice. Shaded bars and open bars correspond to, respectively, control and transgenic mice.

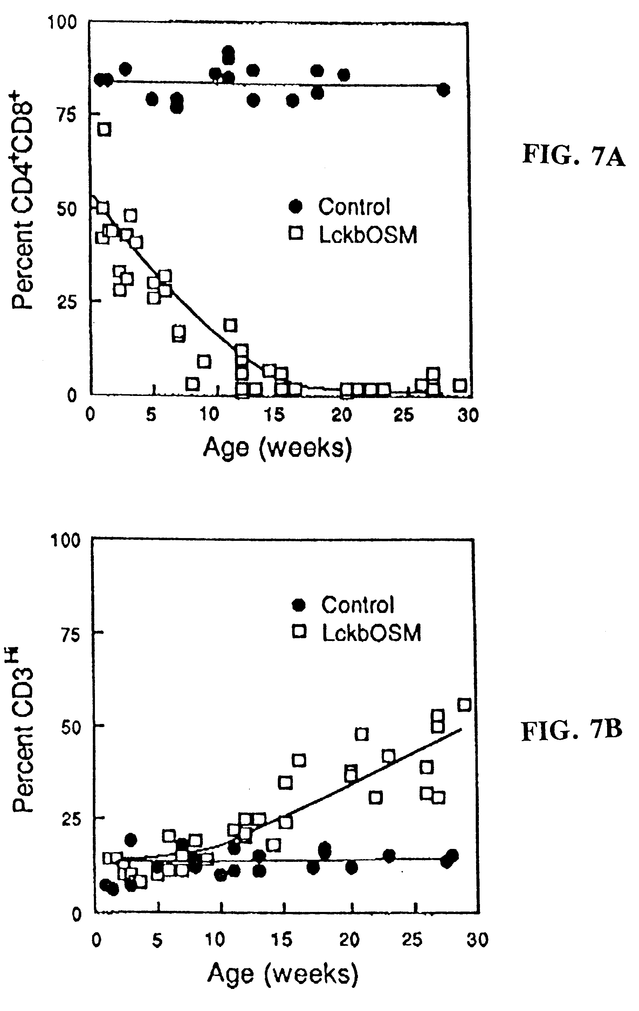

Figs. 7A and 7B show age related changes in lymphocyte profile in the thymus of LckbOSM transgenic mice. (A) The frequency of CD4+CD8+DP "thymocytes"; closed circles are nontransgenic and open squares are LckbOSM transgenic mice. (B) The frequency of mature T-cells as measured by CD3^{hi} staining; closed circles are nontransgenic and open squares are LckbOSM transgenic mice. The frequency of B-cells can be extrapolated from this graph by subtracting the percentage of T-cells from 100%.

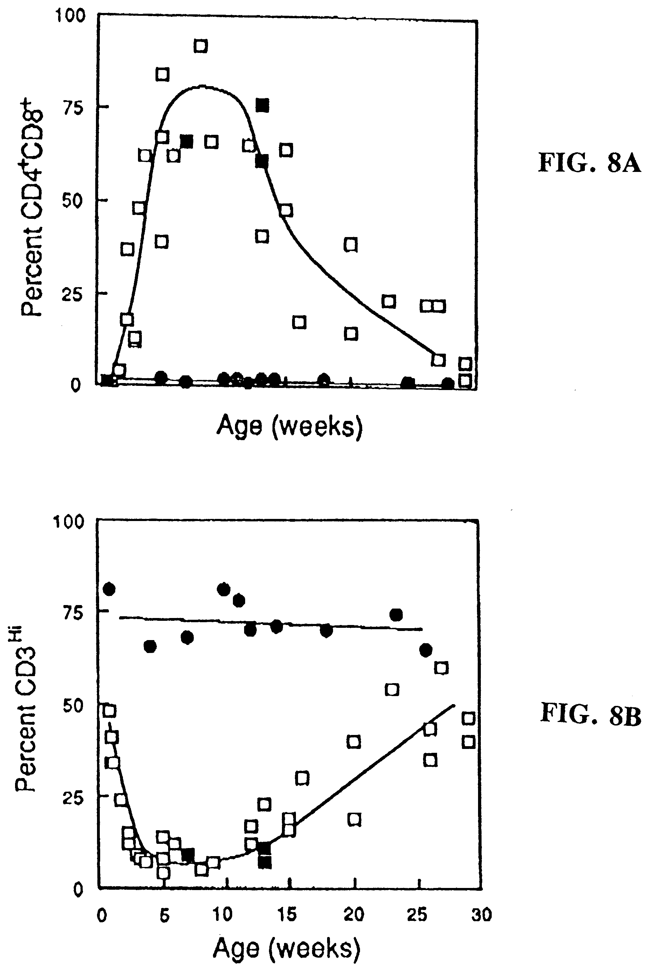

Figs. 8A and 8B show age related changes in lymphocyte profile in the mesenteric node of LckbOSM transgenic mice. (A) The frequency of CD4+CD8+DP "thymocytes". Closed circles are nontransgenic and open squares are LckbOSM transgenic mice. (B) The frequency of mature T-cells as measured by CD3^{hi} staining; closed circles are nontransgenic and open squares are LckbOSM transgenic mice. The frequency of B-cells can be extrapolated from this graph by subtracting the percentage of T-cells from 100%.

### DESCRIPTION OF THE SPECIFIC EMBODIMENTS

The present invention is directed to a method of inducing proliferation and differentiation of mammalian hematopoietic stem cells (hereinafter, "HSCs") by transfection with a vector comprising a gene for oncostatin M. The gene is expressed so as to produce an effective amount of oncostatin M and the induced HSCs are grown in a medium supporting hematopoietic proliferation and differentiation.

In another aspect, the present invention is directed to a method of increasing the survival of lymphocytes. The method comprises transfecting hematopoietic stem cells with a gene coding for oncostatin M and expressing the gene to induce hematopoiesis within a mammal. Inducing hematopoiesis within the mammal increases the survival of the mammal's lymphocytes when exposed to a cytotoxic agent such as radiation or a chemotherapeutic, such as mitomycin C.

The methods of the present invention are useful in the treatment of such diseases as lymphoma, aplastic anemia, β-thalassemia, thrombocytopenia, hemophilia, combined immunodeficiency, AIDS, leukemia. The methods of the present invention are also useful in reducing the lymphocytic depleting effects of radiation therapy or chemotherapy.

Nucleic acids, homologs, and fragments thereof encoding oncostatin M or a fragment thereof (i.e., the oncostatin M "gene") capable of inducing hematopoiesis are disclosed in co-pending applications U.S. Serial Nos. 07/623,867, 08/075,199, 08/078,707, 08/085,279, and 08/312,205, all of which are incorporated herein by reference.

The gene for oncostatin M is introduced in a vector operably linked to a promoter and transcriptional terminator. The choice of promoters will be dictated by the requirement that an effective amount of oncostatin M be expressed to induce HSC hematopoiesis and may be inducible, repressible or constitutive. Further, a promoter may be used so as to exert control over oncostatin M expression after implantation of transfected HSCs into a mammalian host. A variety of tissue-specific promoters may be used to induce differentiation of HSCs in particular lymphatic organs. For example, the thymus specific proximal promoter of the lck gene may be used to drive lymphopoietic differentiation to B-cells and T-cells. Malik et al, Mol. Cell. Biol., 15(5):2349-2358 (1995), incorporated herein by reference. The constructs for modification will normally include a marker which allows for selection of cells into which the DNA has been integrated. Various markers exist, particularly antibiotic resistance markers, such as resistance to G418, hygromycin, and the like. The constructs can be prepared by a variety of conventional ways. Numerous vectors are available which provide various promoters, markers, and restriction sites. Thus, one may introduce the gene for oncostatin M in an appropriate vector along with appropriate transcriptional and translational initiation and termination sites.

HSCs of the present invention are transfected with the gene for oncostatin M either ex vivo (e.g., in cell culture) or in vivo (e.g., within the bone marrow of a mammal). HSCs may be transfected in vivo using, for example, direct retroviral infection or liposome transfection. Nabel et al, Science, 249:1285-1288 (1990). For example, liposomes may be targeted to human HSCs using anti-CD34 antibodies or binding fragments associated with the lipid membrane of HSCs. Typically, HSCs are removed from the mammalian host and transfected ex vivo using such methods as co-precipitation, protoplast fusion, electroporation, or retroviral transfection. See, e.g., J. Sambrook, E.F. Fritsch & T. Maniatis' Molecular Cloning, A Laboratory Manual, Second Edition, Cold Spring Harbor Laboratory Press, 1989, incorporated herein by reference.

The vector used for oncostatin M transfection of HSCs may be chosen to allow for expression of an effective amount of oncostatin M ex vivo, in vivo, or both. Typically, ex vivo expression of oncostatin M transfected HSCs is achieved while the cells are present in a culture medium supporting hematopoietic proliferation and differentiation. Following ex vivo expression, the cells may be implanted in a host and an effective amount of oncostatin M may continue to be expressed once the transfected HSCs are grown in vivo. HSCs may be separated from differentiated cells before in vivo implantation. Alternatively, oncostatin M transfected HSCs are introduced into a host and oncostatin M is expressed in vivo (e.g., using a tissue specific promoter) to produce an effective amount of the gene product. As will be understood by those of skill in the art, the host should be immunotolerant of implanted HSCs. For example, the host may receive immunosuppressants or be of sufficient histocompatibility so as to avoid a graft rejection. An effective amount of oncostatin M will induce hematopoietic proliferation and differentiation. Methods of assaying for hematopoietic proliferation and differentiation are known in the art.

After inducing expression of an effective amount of oncostatin M, HSCs are grown in an ex vivo or in vivo medium to permit HSC proliferation and differentiation. The medium in which HSCs are grown may be ex vivo, as in, for example, a culture medium, or in vivo, for example, in bone marrow of a host. In many situations, prior to introduction of stem cells, the host may be treated to ablate native lymphocytic or hematopoietic capability. Oncostatin M transfected HSCs may be administered in any physiologically acceptable medium, normally intravascularly, although they may also be introduced into the bone cortex or other convenient site where the cells may find an appropriate site for regeneration and differentiation. Usually, at least about 1 X 10³ cells will be administered, preferably 1 X 10⁴ or more. The cells may be introduced by injection, catheter, or the like. If desired, depending upon the purpose of introducing the cells, factors may be introduced such as interleukins, e.g., IL-2, Il-3, IL-6, G-CSF, IFN-γ, erythropoietin, thrombopoietin, etc. The amount of these factors will depend upon the purpose for administering the cells and the particular needs of the patient.

HSCs of the present invention may be used in gene therapy, directed to differentiate to a particular lineage or subset of such lineage, or enhanced for a particular function. For example, rearranged T-cell receptors or immunoglobulin genes may be introduced into HSCs which are directed to lymphopoietic differentiation by driving oncostatin M expression from the thymus specific proximal lck gene promoter. The resulting B-cells and T-cells would be expected to express Igs and TCRs, respectively, specific to a selected epitope.

In another embodiment, oncostatin M transfected HSCs may also be stimulated to overexpress double-positive cells expressing CD4 (e.g., CD4⁺-CD8⁺) by driving lymphocytic differentiation with promoters such as the proximal lck gene promoter. These double-positive cells may be used as a sink to reduce HIV viral load and thereby protect mature CD4⁺ T-cells. Co-transfection of double-positive cells with specific dominant-negative mutations for viral infection may be used to inhibit subsequent infection.

In another embodiment, lymphopoiesis is stimulated in individuals in which thymus derived T-cell development is impaired or lacking, that is, thymectomized or athymic. Lymphopoiesis may be stimulated in such individuals using a thymus specific promoter such as the proximal lck gene promoter to drive expression of oncostatin M in transfected HSCs.

In another aspect of the invention, an effective amount of oncostatin M is used to increase the survival of lymphocytes. The lymphocytes which are rescued from the effects of cytotoxic agents (e.g., chemotherapeutics, radiation) may be non-transfected host lymphocytes or transfected lymphocytes derived from oncostatin M transfected HSCs. In accordance with the methods of the present invention, a variety of combinations of expression and growth of oncostatin M transfected HSCs may be employed. For example, oncostatin M of transfected HSCs may be expressed ex vivo to expand the culture to a desired level and, subsequently, introduced in vivo for continued expression and growth in the medium of host bone marrow. Alternatively, an effective amount of oncostatin M may be both expressed and grown in vivo using, for example, a tissue specific promoter operably linked to the gene for oncostatin M. HSCs undergoing oncostatin M induced hematopoiesis are less susceptible to the cytotoxic activity of radiation and chemotherapy. Thus, for example, oncostatin M induced HSCs may be utilized to lessen the reduction in host lymphocyte levels resulting from administration of the cytotoxic agent, mitomycin C. Additionally, oncostatin M induced HSCs are themselves more tolerant of cytotoxic agents than non-transfected (non-induced) HSCs. Induction of hematopoiesis by expression of an effective amount of oncostatin M may be used prior to, during, or after treatment with a cytotoxic agent. Preferably, induction occurs during or after treatment.

The following examples are offered by way of illustration, not by way of limitation.

### Example I

This Example describes cloning, expression and analysis of bovine oncostatin M.

One hundred five plaques of a bovine genomic library (Stratagene) were screened with the ³²P-labelled *Bg*lII-*Xho*I fragment of human oncostatin M (hOSM) cDNA. Nitrocellulose filters were hybridized overnight at 42°C in hybridization buffer containing 50% formamide and washed in 2 X SSC (1 x SSC is 0.15 M NaCl plus 0.015 M sodium citrate)-0.1% sodium dodecyl sulfate (SDS) at 65°C and then in 0.5x SSC-0.1% SDS at 65°C. Purified λ phage DNA was subjected to Southern hybridization analysis, and a 4.7-kb *Eco*RI fragment was subcloned into Bluescript II SK⁺ (Stratagene). The open reading frame and exon/intron boundaries of the bOSM gene were sequenced from both strands by the dideoxy-chain termination method. The bOSM gene was expressed in cells by using a 3.2-kb *Sst*I-*Eag*1 fragment. This DNA begins 12 bp upstream of the initiator Met and continues 340 bp past the termination codon. An AT-rich sequence thought to mediate rapid turnover of various cytokine mRNAs (Shaw et al., Cell, 46:659-667 (1986), incorporated herein by reference) lies downstream of the distal *Eag*1 site and was not included in this genomic fragment. The 3.2-kb bOSM DNA was treated with Klenow fragment and cloned by blunt-end ligation into pπH3 (Aruffin et al. Proc. Natl. Acad. Sci. USA, 84:8573-8577 (1987), incorporated herein by reference), which contains the cytomegalovirus promoter and simian virus 40 origin of replication. Cloning of the human OSM gene in pπH3 and its expression in COS cells are as described in Malik et al., Mol. Cell. Biol., 9:2847-2853 (1989), incorporated herein by reference. COS cells were cultured in Dulbecco's modified Eagle's medium containing 10% fetal bovine and transfected with DEAE-dextran sulfate. After 48 h. cultures were fed serum-free medium, and this conditioned medium was collected 24 h later. For Western immunoblot analysis, conditioned medium was dialyzed against 1 M acetic acid and subjected to SDS-polyacrylamide gel electrophoresis. Proteins were transferred onto nitrocellulose and incubated with rabbit antisera raised against a bOSM peptide (RTAGQVLRGWGERQGRSRRC). The bOSM-antibody complex was detected with alkaline phosphatase-conjugated protein A.

One positive clone was analyzed by Southern hybridization and subjected to DNA sequence analysis. Fig. 1 shows a partial restriction map of a 4.7-kb *Eco*RI fragment encoding a protein related to hOSM. The open reading frame for this gene is contained on three exons and is 73% identical to that of hOSM. Introns I and II are 1,418 and 572 nucleotides in length, respectively.

Fig. 2 presents a model of the hOSM protein and the alignment of the bOSM amino acid sequence with the human and simian proteins (Bruce et al., Prog. Growth Factor Res., 4:157-170 (1992), incorporated herein by reference). The exon/intron borders dividing the open reading frames of all three genes are identical. The bovine and human precursor proteins are 58% identical. Significant similarities can be observed in various regions, including the signal peptide and the carboxyl extension which is removed from hOSM by proteolysis. The hOSM protein is thought to contain four amphipathic helices with a repeated pattern of apolar residues occurring in the *i* and *i*+3 positions of a 7-residue heptad. Many of the apolar residues with the four helices of bOSM are identical with those in hOSM, and many of the substitutions represent conservative changes. The four cysteine residues involved in disulfide bonding are also identical, and two regions thought to be important for receptor binding, the AB loop and the D1 region, are strongly conserved. One notable exception in the D1 region is a G-to-N change. This G residue is prominent in helical turns and is conserved in LIF, CNTF, and granulocyte colony-stimulating factor. Another change that potentially alters the helical bundle relationship in the bovine protein is a 14-amino-acid deletion that includes the loop region following helix B and the beginning of helix C. This divergence in sequence may not be critical for function, since helices B and C are thought to lie opposite the receptor-binding domain.

### Example II

This Example describes a comparison of human and bovine oncostatin M in a receptor binding and a growth inhibition assay.

In the oncostatin M (OSM) receptor-binding essay, a 7-ml portion of conditioned medium containing recombinant bOSM (equivalent to 630 ng of hOSM as determined by growth inhibition assays) was lyophilized and resuspended in 1 ml of binding buffer (20 mM *N*-hydroxyethylpiperazine-*N*'-2-ethanesulfonic acid [HEPES; pH 7.4), 5% bovine serum albumin BSA, and 0.2% sodium azide in RPMI 1640) that included 500 pM[¹²⁵I]hOSM. This cocktail was diluted in 2-fold increments with binding buffer containing [¹²⁵I]hOSM to a final dilution of 128-fold. H2981 lung carcinoma cells (Horn et al., Growth Factors, 22:157-165 (1990), incorporated herein by reference) were cultured (75,000 cells per well) in Dulbecco's modified Eagle's medium plus 10% fetal bovine serum for 24 h. The cells were washed once with binding buffer and then incubated in diluted conditioned medium (200 µl) for 1.5 h at 20°C on a rotary shaker. The cells were washed three times with 500 µl of binding buffer, solubilized in 200 µl of 1 M NaOH at 60°C for 30 min. and then assayed for bound radioactivity with a Packard Cobra Autogamma counter. Each datum point represents the average and standard deviation from triplicate determinations (Fig. 3).

In the growth inhibition assays, recombinant bOSM was assayed by previously described methods for the ability to inhibit the proliferation of A375 melanoma cells. Malik et al, Cell Biol., 11:453-459 (1992); Rose et al, Proc. Natl. Acad. Sci. USA, 88:8641-8645 (1991), both incorporated herein by reference. Briefly, 3.5 x 10⁶ cells were seeded per well in a 96-well microtiter plate containing Dulbecco's modified Eagle's medium plus 10% fetal bovine serum. After 2 h, the medium was replaced with 200 µl containing dilutions of conditioned medium with bOSM protein. At 72 h after plating, cultures were pulsed for 8 h with 100 µl of fresh medium containing ¹²⁵I-deoxyuridine (0.1 µCi). Cells were then lysed with 1 M NaOH, and the amount of radioisotope incorporated into DNA was determined. Mouse M1 myeloid leukemia cells (2.5 x 10³ per well) were incubated for 72 h in RPMI 1640 medium containing 10% fetal bovine serum and in various dilutions of conditioned medium containing bOSM protein. The cells were pulsed for 6 h with 50 µl of medium containing 0.1 µCi of [³H]thymidine and were then harvested, and the counts per minute incorporated into DNA was determined. Each datum point represents the mean of triplicate determinations (Figs. 4 & 5).

To determine whether the bOSM gene encodes a biologically active protein, we tested its ability to bind receptor and function on cultured cells. A 3.2-kb portion of the bOSM gene with its open reading frame and two introns was inserted into pπH3, an expression plasmid containing the cytomegalovirus promoter and the simian virus 40 origin of replication. COS cells were transfected and incubated in serum-free medium. Supernatants were then assayed for bOSM protein by Western blot analysis with a rabbit anti-serum raised against a bOSM peptide. A protein with the expected size of about 30 kDa was detected in bOSM-transfected cells but not in mock-transfected cultures.

To test whether hOSM competes with bOSM for receptor binding, we incubated human H2981 lung carcinoma cells with [¹²⁵I]hOSM and various dilutions of protein prepared from either bOSM-transfected or mock-transfected COS cell supernatants. Ninety minutes later cultures were rinsed, lysed, and measured for [¹²⁵I]hOSM binding. As shown in Fig. 3, medium containing the bOSM protein prevented 85% of hOSM from binding to cells, a level similar to that obtained with excess unlabeled hOSM. Supernatants from control COS cells had little effect on hOSM binding. This result suggests that bOSM can recognize the OSM-specific receptor on human cells, since H2981 cells do not express LIFRβ or bind LIF.

A375 cells, derived from a human melanoma, became round and stopped growing in the presence of nanomolar levels of hOSM. This inhibition occurs through the OSM-specific receptor. Mouse M1 leukemia cells also stopped dividing in the presence of hOSM and differentiated into a quiescent macrophage-like cell. This effect occurred through the receptor complex that contains gp130 and LIFRβ. To test the functional activity of bOSM on these two cell types, supernatants from bOSM-transfected COS cells and control transfected cells were diluted in culture medium containing 10% serum. A375 cells (Fig. 4) and M1 cells (Fig. 5) were incubated in the presence of various dilutions for 3 days. As indicated, DNA synthesis was inhibited in both cell lines in a dose-dependent manner. Control experiments with dilutions of supernatants from mock-transfected cells had no effect on cell proliferation. These results, in combination with the receptor competition assay described above, strongly suggest that the gene isolated from the bovine genomic DNA library encodes a biologically active protein that can interact with both mouse and human gp130-based receptors.

### Example III

This Example describes targeted expression of bovine oncostatin M in transgenic mice.

All transgenes contained the 3.2-kb bOSM genomic fragment (Example I). Expression vectors and their regulatory sequences were as follows: MtbOSM, which contained the 0.7-kb mouse metallothionein promoter and the 3' polyadenylatlon signal sequence (0.63 bp) from the human growth hormone gene Low et al, Cell, 41:211-219 (1985), incorporated herein by reference; RIβbOSM, a 3.5-kb promoter fragment of the RIβ subunit of cyclic AMP (cAMP)-dependent protein kinase (Rogers et al., Mol. Endocrinol., 6:1756-1765 (1992), incorporated herein by reference) and the 3' intron and polyadenylation signal sequence (0.5 kb) from the mouse protamine-1 (mP1) gene (Peschon et al, Proc. Natl. Acad. Sci. USA, 84:5316-5319 (1987), incorporated herein by reference); LekbOSM, the 3.1-kb proximal promoter of the lck gene (Chaffin et al., EMBO J., 9:3821-3829 (1990), incorporated by reference) and the 0.5-kb mP1 DNA; K14-bOSM, 2.7 kb of the mouse keratin-14 gene promoter (Vassar et al., Genes Dev., 5:714-727 (1991), incorporated herein by reference) and the 0.63-kb human growth hormone DNA; and InsbOSM, the 0.6-kb promoter and noncoding exon 1 of the rat insulin-1 gene (Lo et al., Cell, 53:159-168 (1988), incorporated herein by reference) and the 0.63-kb human growth hormone DNA. Transgenes were separated from plasmid sequences following restriction enzyme digestion, agarose gel electrophoresis, and elution into TAE (0.04 M Tris-acetate [pH 8.4], 0.001 M EDTA) buffer. The DNA was resuspended (10 µg/ml) in low TE (10 mM Tris [pH 7.5], 0.5 mM EDTA) and filtered through a prerinsed 0.22 µm-pore-size acrodisk (Gelman Sciences). Fertilized oocytes from (C3H x C57BL/6)F, mice were used for pronuclear injections. Embryos were cultured overnight and then transferred into the oviducts of pseudopregnant C57BL/6 females. Transgenic mice were identified following hybridization of tail DNA with radiolabelled DNA probes. Tissues prepared for necropsy were fixed in 4% paraformaldehyde and embedded in paraffin. Selected material was stained with hematoxylin and eosin and then analyzed microscopically.

Human OSM regulates a range of biological responses in cultured cells, but very little is known about its effects in vivo. Accordingly, a series of experiments designed to target bOSM expression in transgenic mice was undertaken. Table 1 (below) lists the various tissue-specific promoters used to regulate the bOSM gene and the frequency of transgenic mice obtained with these and other nonrelated transgenes.

**TABLE 1**

| Targeted Expression of bOSM in Transgenic Mice | | | | | |
|---|---|---|---|---|---|
| Promoter | Tissue | Gene | No. of pups born | No. of transgenic pups | % of transgenic pups |
| Mt-1 | Ubiquitous | Osteoglycin | 51 | 12 | 23.5 |
| | | Epithelin | 41 | 8 | 18.1 |
| | | bOSM | 116 | 1 | 0.8 |
| Keratin-14 | Basal Cell | H3 | 93 | 13 | 17.3 |
| | Epithelia | Amphiregulin | 75 | 9 | 9.6 |
| | | bOSM | 136 | 0 | 0 |
| RIβ | Neurons | RIβ-LacZ | 50 | 10 | 20.0 |
| | | | 219 | 16 | 7.3 |
| Insulin-1 | β cells | NGF* | 47 | 8 | 17.0 |
| | | bOSM | 73 | 3 | 4.1 |
| Lck | Thymocytes | bOSM | 55 | 2 | 3.6 |

| | | | | | |
|---|---|---|---|---|---|
| *NGF, nerve growth factor | | | | | |

The initial test for identifying organ systems that may respond to bOSM utilized the metallothionein promoter, which directs transcription in numerous tissues. The frequency of bOSM transgenic mice obtained with this promoter was significantly reduced in comparison with the frequency obtained when using the osteoglycin or epithelin genes, suggesting that expression of bOSM in multiple organs is deleterious during mouse development. This argument is strengthened by the phenotypes displayed by the lone transgenic animal (TG 943) that was mosaic for MtbOSM; the transgene was intact as determined by Southern analysis, but the mouse contained less than one copy of the transgene per cell. This mouse displayed a chronic tremor and had grossly enlarged hind limbs. Efforts to breed this male animal failed. Following necropsy, it was determined that spermatogenesis was defective in this mouse; no mature sperm were detected in the ducts of the epididymis, nor were developing spermatocytes apparent in the seminiferous tubules. Spermatogonia which line the periphery of the tubule were absent. The enlarged hind legs of TG 943 resulted from excessive growth of bone in the femur and phalanges. The marrow cavities were filled with immature bone and new periosteal bone deposition. Closer examination revealed that the growth plate of the femur was disrupted and disorganized. The haversian system was present but minimal, and very little reorganization, if any, occurred within this new bone.

Because OSM is a potent mitogen for AIDS-related Kaposi's sarcoma cells, the keratin-14 (K14) promoter was used to derive a mouse that expressed bOSM in the basal layer of stratified epithelium. The frequency of transgenic mice obtained with this promoter and an unrelated gene, βH3, was nearly 20%. As a result of severe keratinosis, the frequency of transgenic mice expressing mouse amphiregulin a ligand for the epidermal growth factor receptor, was reduced to approximately 10%. However, no transgenic animals were recovered when K14-bOSM was used, suggesting that expression of bOSM within developing skin is lethal.

As with LIF and CNTF, OSM induces neuropeptide gene expression in neuroblastoma cells. To test the potential effects of bOSM on neurons in vivo, the promoter for the RIβ subunit gene of cAMP-dependent protein kinase, which directs neuron-specific gene expression within the brain, spinal cord, and peripheral ganglia. The transgenic bOSM frequency (7.3%) was approximately three times lower than normal. Five animals, all runted in comparison with littermates, experienced severe tremors with ataxia. These animals displayed a progressive weakness, initiating with the hind limbs, and died within 6 to 8 weeks. The remaining four animals showed no phenotype and no transgene expression. These results suggest that expression of bOSM in neurons was detrimental, but the inability to establish a viable mouse line with this promoter precluded the analysis of bOSM action in nerve.

The frequency of bOSM transgenic mice obtained with the rat insulin-1 promoter was also reduced significantly. A line of animals was established with this construct that showed consistent bOSM mRNA expression in pancreatic islet cells. These mice demonstrated massive stromal cell proliferation around islets and blood vessels. This fibrosis was accompanied by acinar atrophy and edema. Most islets appeared normal within the stroma, although some were surrounded by large cuffs of lymphocytes. Hyperglycemia was not observed in these mice, suggesting that islet function was not compromised. Possibly because of an accumulation of bOSM in serum, it was found that the number of megakaryocytes in the bone marrow cavities in numerous InsbOSM animals was increased more than 10-fold. This result is consistent with the finding that OSM stimulates megakaryocyte colony formation in vitro and platelet production in vivo. Vassar et al., supra.

Finally, bOSM was tested for whether it could influence T-cell development by targeting its expression to the thymus by using the proximal lck promoter. Animals within the LckbOSM lineage acquired a lethal autoimmune phenotype and significant changes in lymphoid tissue development. For instance, the thymus architecture was disrupted and the demarcation of cortical and medullary regions was lost. The major cell type normally found within the cortex, CD4⁺ CD8⁺ thymocytes, was dramatically underrepresented, and these regions were supplanted with follicles containing B lymphocytes. In addition to the presence of splenomegaly, the nodes became enlarged because of a lymphoproliferation of mostly CD4⁺CD8⁺ "thymocytes." Thus, the normal separation of primary (thymus) and secondary (node) lymphoid tissues is lost in these animals, and this may contribute to the autoimmune phenotype.

### Example IV

This Example describes the development and the characterization of lymphoid tissue of transgenic mice expressing human and bovine oncostatin M under control of the proximal Lck promoter.

Transgenic mice were created per the methods described in Example III. Fertilized oocytes from (C3H X C57Bl/6) F₁ mice were used for pronuclear injections. Embryos were cultured overnight and then transferred into the oviducts of pseudopregnant C57BL/6 females. Transgenic mice were identified following hybridization of tail DNA with radiolabeled probes.

Oncostatin M (OSM) expression in LckbOSM transgenic mice caused a dramatic increase in the size and weight of lymphoid tissue, including thymus, spleen, and peripheral nodes. Cellularity of transgenic lymph nodes was found to increase up to fifty-fold compared to control tissue. Cellularity of the spleen and thymus increased due to lymphoproliferation. However, splenomegaly of transgenic animals is not the result of lymphoproliferation. Instead, the results suggested that the spleen, as well as other lymphoid tissue, contained increased numbers of myeloid and erythroid stem cells. In contrast to spleen and mesenteric nodes, cellularity of transgenic thymuses decreased relative to non-transgenic controls.

The various cell types present in thymus, spleen, and lymph nodes was determined by FACS analysis and compared to non-transgenic mice. As shown in Fig. 7A, in contrast to control thymuses, CD4⁺CD8⁺ (DP) thymocytes were undetectable in transgenic mice after 10 weeks of age. Instead, B-cell lymphopoiesis was detected. Additionally, an accumulation of "mature" T cells was detected as evidenced by an increase in cells displaying high levels of CD3 (Fig. 7B). Thus, the transgenic thymus acquired a mixed phenotype of bone marrow and secondary lymphoid tissue. In contrast to LckbOSM transgenics, autoimmunity was absent or significantly reduced in reconstituted mice.

While the cellularity of transgenic lymph nodes was greatly increased, the ratio of T- and B-cells in these tissues was found to be fairly normal, indicating that oncostatin M had lymphopoietic activity on both these cell types. However, the majority of cells in the transgenic node resembled CD4⁺CD8⁺ (DP) thymocytes (i.e., HSA⁺, CD₃^{dull}, MHC I^{dull}). As shown in Fig. 8A, these cells first appeared 10 days after birth, increased in cell frequency to nearly 60% at 10 weeks, and gradually disappeared by 30 weeks of age. A concomitant increase was seen for "mature" T-cells (Fig. 8B). Thus, the nodes became permissive for T-cell lymphopoiesis.

In sum, LckbOSM mice showed increased T- and B-cell lymphopoiesis, the locations of which were abnormal. Additional data indicated that OSM stimulated the proliferation of stem cells within other hematopoietic lineages. The dramatic increase in DP cells and B-cells in peripheral nodes indicates that OSM acts as an autocrine growth factor.

### Example V

This Example describes identification of the origin of node thymocytes and the ability of transgenic bone marrow to rescue host lymphocytes.

Bone marrow was isolated from LckbOSM mice by and 1 X 10⁷ bone marrow cells were injected into the tail vein of normal, thymectomized, and athymic nude mice which were lethally irradiated (1000 rads). As indicated in Table 2 (below), only mice reconstituted with OSM bone marrow contained double-positive cells typifying the normal thymus. In all cases this OSM phenotype was transferred into the mesenteric nodes of mice irrespective of whether they possessed a thymus, were thymectomized, or were athymic. Thus, OSM expression stimulated prothymocyte proliferation and differentiation independent of the normal thymus environment.

**TABLE 2**

| Reconstitution of Mesenteric Node | | | | | | | | |
|---|---|---|---|---|---|---|---|---|
| Donor BM | Untreated | Control | Control | OSM | OSM | Untreated | Control | OSM |
| Host Mouse | Control (5) | +Thy(5) | -Thy(2) | +Thy(4) | -Thy (4) | Nu(1) | Nu(2) | Nu(2) |
| Relative Cell No. | 1 | 0.2 | 0.5 | 9 | 2.6 | -- | -- | -- |
| Relative Cell No. | -- | --- | --- | -- | --- | 1 | 3.5 | 17.5 |
| % CD+4+CD8+ | <1.0 | <1.0 | <1.0 | 42 | 35 | <1.0 | <1.0 | 59 |
| Host mice received lethal irradiation and were reconstituted with the indicated donor bone marrow (BM). Host mice were normal B6/C3H F1 mice (+Thy), thymectomized B6/C3H F1 mice (-Thy) and B6 Nude mice (Nu). Donor bone marrow was from control B6 mice or LckbOSM mice. Animals, numbers of which are indicated in parentheses, were examined beginning 6-7 weeks after treatment. | | | | | | | | |

The adoptive-transfer experiments also demonstrated that LckbOSM bone marrow effectively rescued host lymphocytes. The host B6/C3H F₁ mice expressed both H2-D^{b} and H2-D^{k} haplotypes whereas the B6 donor expressed only the H2-D^{b} haplotype. The vast majority of lymphocytes in mice reconstituted with control bone marrow expressed the H2-D^{b} haplotype. The cells in mice reconstituted with OSM bone marrow were a mixture between H2-D^{b}, H₂-D^{bk}, and dull b, the latter of which is expected for DP thymocytes. This result suggested that host derived bone marrow/lymphocytes were rescued by OSM bone marrow and that OSM rescues host cells that would normally have died from irradiation.

All publications, patent applications and patents mentioned in this specification are herein incorporated by reference into the specification to the same extent as if each individual publication or patent was specifically and individually indicated to be incorporated herein by reference.

Although the foregoing invention has been described in some detail by way of illustration and example for purposes of clarity of understanding, it will be obvious that certain changes and modifications may be practiced within the scope of the appended claims.

## Claims

1. A method of inducing proliferation and differentiation of mammalian hematopoietic stem cells, comprising:
transfecting said stem cells with a vector comprising a gene coding for oncostatin M;
expressing said gene to produce an effective amount of oncostatin M; and
growing said stem cells in a medium supporting hematopoietic proliferation and differentiation.

2. The method of claim 1, wherein said cells are grown in a histocompatible mammalian host.

3. The method of claim 1, wherein said vector is a retroviral vector.

4. The method of claim 1, wherein said gene is under the control of a constitutive or tissue specific promoter.

5. The method of claim 4, wherein said promoter is the proximal Lck promoter.

6. The method of claim 5, wherein said stem cells are grown in bone marrow.

7. The method of claim 6, wherein said stem cells further comprise a vector expressing a rearranged T cell receptor or immunoglobulin.

8. The method of claim 1, wherein said stem cells are grown *ex vivo*.

9. The method of claim 8, wherein after ex vivo growth said cells are implanted in a mammalian host.

10. The method of claim 2, wherein said host is thymectomized or athymic.

11. A method of increasing the survival of lymphocytes, comprising:
transfecting hematopoietic stem cells with a gene coding for oncostatin M; and
expressing said gene to induce hematopoiesis within a mammal, wherein said expression increases the survival of said lymphocytes within said mammal to cytotoxic agents.

12. The method of claim 11, wherein the host is irradiated to eliminate the hematopoietic system.

13. The method of claim 11, wherein said cytotoxic agents are chemotherapeutic drugs or radiation.

14. The method of claim 13, wherein said chemotherapeutic drug is mitomycin C.

15. The method of claim 11, wherein said lymphocytes are derived from said transfected stem cell.

16. The method of claim 11, wherein said lymphocytes are non-transfected lymphocytes of said mammal.
